# EUROPEAN PATENT SPECIFICATION

(11) **EP 2 056 926 B1**
(45) Date of publication and mention of the grant of the patent: **25.08.2010**
(21) Application number: 07710265.5
(22) Date of filing: 23.01.2007
(51) Int. Cl.: A61N 1/05

(54) **NOVEL FEATURES FOR ROUTING CONDUCTORS IN MEDICAL ELECTRICAL LEAD ELECTRODE ASSEMBLIES**
NEUE MERKMALE FÜR ROUTING-LEITER IN MEDIZINISCHEN ELEKTROLEITUNG-ELEKTRODEN-ANORDNUNGEN
NOUVELLES CARACTÉRISTIQUES POUR ACHEMINER DES CONDUCTEURS DANS DES ENSEMBLES ÉLECTRODE FIL CONDUCTEUR ÉLECTRIQUE MÉDICAUX

(30) Priority: 21.08.2006 US 465879; 21.08.2006 US 465941; 21.08.2006 US 465946
(43) Date of publication of application: 13.05.2009
(73) Proprietor: Medtronic, Inc., Minneapolis MN 55432-5604 (US)
(72) Inventor: SKUBITZ, Sean, P., Forest Lake, MN 55025 (US); BOATWRIGHT, Mary, L., Andover, MN 55304 (US); BOLEA, Stephen, L., Watertown, MN 55388 (US); TOWER, Jessica, L., Wauwatosa, WI 53213 (US); METZLER, Michael, E., Minneapolis, MN 55408 (US)
(74) Representative: Hughes, Andrea Michelle
(86) International application number: PCT/US2007/060888
(87) International publication number: WO 2008/024526

(56) References cited:
- US-A- 3 738 368
- US-A1- 2005 090 885

## Description

### TECHNICAL FIELD

The present invention is related to medical electrical leads and more particularly to electrode assemblies thereof.

### BACKGROUND

Medical electrical leads include one or more conductors that extend within an elongate insulative body and are coupled to one or more electrodes supported by the body. The one or more electrodes are typically mounted to a distal portion of the lead body and the distal portion positioned, or implanted, in a patient's body to provide electrical stimulation, for example, within a pericardial space, to provide restorative cardiac stimulation, or, within an epidural space, to provide pain-relieving spinal stimulation.

The portion of the lead body that supports the one or more electrodes should be configured to, at minimum, allow each electrode surface to make contact with a target stimulation site, support each joint between the one or more electrodes and the corresponding conductor, and, in the case of more than one electrode, electrically isolate the electrodes and conductors from one another. Electrode assemblies have been developed, for example, within the context of the exemplary stimulation scenarios referenced above, wherein a 'flattened', or relatively thin, lead body portion, for example, having a patch or paddle configuration, supports one or more electrodes, preferably an array of electrodes, that are disposed along a major surface of the lead body portion. However there is still a need for electrode assembly features that improve the routing of conductors from the electrodes. Document US 2005/0090885 discloses a medical electrical lead electrode assembly according to the preamble of claim 1.
The present invention provides a medical electrical lead electrode assembly as defined in claim 1.

### BRIEF DESCRIPTION OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.

Figure 1A is a plan view of an exemplary medical electrical lead, according to some embodiments of the present invention.

Figure 1B is an end view of the lead shown in Figure 1A.

Figure 2A is a perspective view of a conductive component, according to some embodiments of the present invention.

Figure 2B is a plan view of a conductive component, according to some embodiments of the present invention.

Figure 3A-B are section views taken through section line D-D of Figure 1A, according to some alternate embodiments of the present invention.

Figure 3C is a perspective view of a conductive component, according to an alternate embodiment of the present invention.

Figure 4A is a perspective view of an insulative carrier showing a first side thereof, according to some embodiments of the present invention.

Figure 4B is a perspective view of an insulative carrier showing a second side thereof, according to some embodiments of the present invention.

Figure 4C is an enlarged view of a portion of the carrier shown in Figure 4B.

Figure 4D is an end view of the carrier shown in Figures 4A-C.

Figure 5A is a plan view of a portion of an electrode assembly, according to some embodiments of the present invention.

Figure 5B is a plan view of a portion of an electrode assembly, according to some alternate embodiments of the present invention.

Figure 5C is a plan view of a portion of an electrode assembly, according to yet further alternate embodiments.

Figures 6A-B are perspective views of a conductive component, according to further alternate embodiments of the present invention.

Figures 7A-B are section views taken through section line D-D of Figure 1A, according to further alternate embodiments of the present invention.

Figure 8 is a flow chart outlining methods of the present invention.

### DETAILED DESCRIPTION

The following detailed description is exemplary in nature and is not intended to limit the scope, applicability, or configuration of the invention in any way. Rather, the following description provides practical illustrations for implementing exemplary embodiments of the present invention. Examples of constructions, materials, dimensions, and manufacturing processes are provided for selected elements, and all other elements employ that which is known to those of skill in the field of the invention. Those skilled in the art will recognize that many of the examples provided have suitable alternatives that can be utilized.

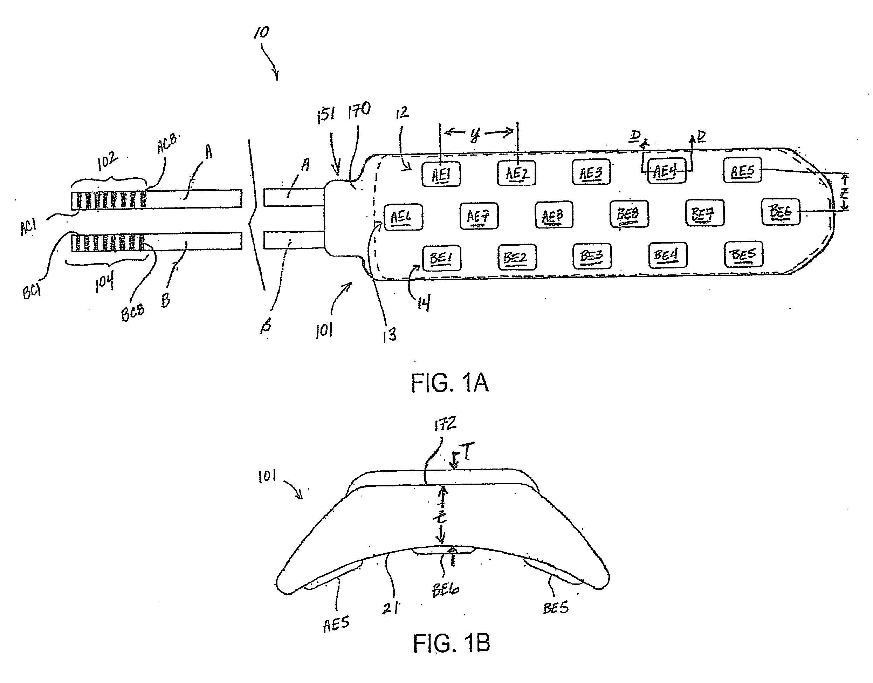

Figure 1A is a plan view of an exemplary medical electrical lead 10, according to some embodiments of the present invention; and Figure 1B is an end view of lead 10. Figure 1A illustrates lead 10 including a pair of elongate insulative tubular bodies A and B which are terminated at a proximal end by connectors 102 and 104, respectively, and which extend distally to terminate in an insulative paddle-shaped body 101 supporting an array of electrodes AE1-8 and BE1-8, which are arranged in three columns 12, 13, and 14. Figure 1B illustrates a profile of body 101, which has a maximum thickness T of approximately 0.009 inch (2.286 mm) along a proximal portion 151 thereof, and a maximum thickness t of approximately 0.075 inch (1.905 mm) along a length thereof that extends distally from proximal portion and corresponds with an extent of columns 12, 13, 14. The shape and profile of body 101 makes lead 10 suitable for epidural implantation to provide spinal cord stimulation; and the arrangement of electrodes AE1-8 and BB1-8 can provide flexibility for selection of a stimulation pattern from a variety of stimulation patterns after lead 10 is implanted, without having to physically reposition lead 10.
According to an exemplary embodiment of the present invention, each electrode in columns 12, 13 and 14 are spaced apart from one another, along a length of each column, by a distance x, center-to-center, which is approximately 0.120 inch (3.048 mm), and each column 12, 13, 14 is spaced apart from one another by a distance z, center-to-center, which is approximately 0.179 inch (4.5466 mm). Although the electrode array illustrated herein provides a suitable example for preferred embodiments of the present invention, it should be noted that various alternate embodiments of the present invention include any number of electrodes in any arrangement.

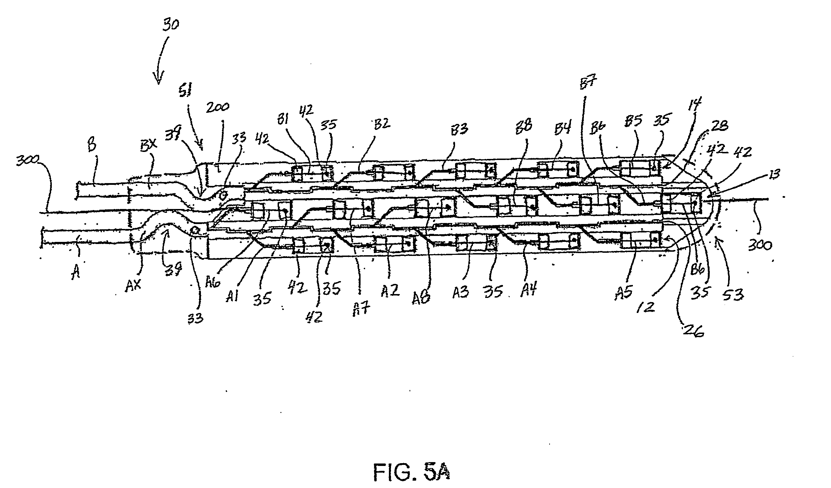

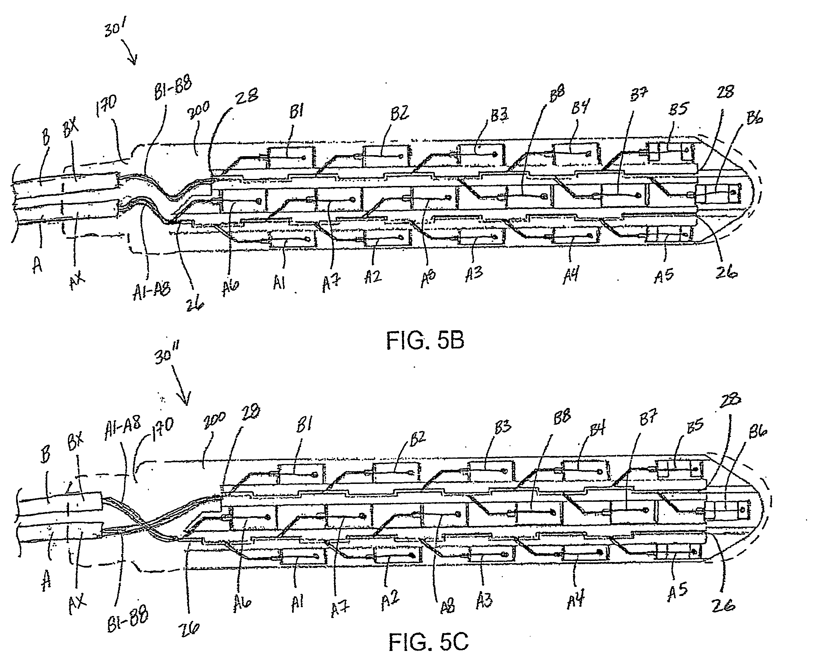

According to the illustrated embodiment, electrodes AE1-8 are coupled to corresponding contacts AC 1-8 of connector 102 and electrodes BE1-8 are coupled to corresponding contacts BC1-8 of connector 104, such that each electrode may be independently powered when the connectors 102, 104 are plugged into a stimulation device. Although not shown, those skilled in the art will appreciate that conductors A1-8 (Figure 5), which couple each of electrodes AE1-8 to a corresponding contact of connector 102, extend within one or more longitudinally extending pre-formed channels, or lumens, of tubular body A, and conductors B1-8 (Figure 5), which couple each of electrodes BE1-8 to a corresponding contact of connector 104, extend within a similar one or more channels, or lumens, of tubular body B. Each of conductors A1-8 and B1-8 may be in the form of a coil or cable, being formed, for example from MP35N alloy, and each preferably includes an insulating jacket extending thereover, being formed, for example, from a fluoropolymer; such conductors are well known to those skilled in the art of medical electrical leads. A routing of the conductors within body 101, according to one embodiment of the present invention, will be described below, in conjunction with Figure 5.

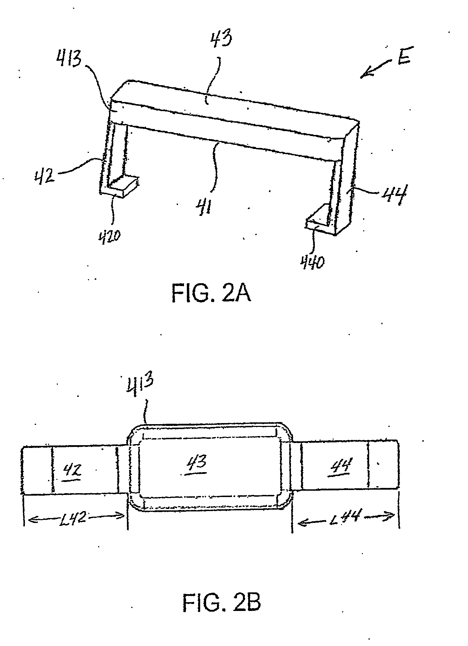

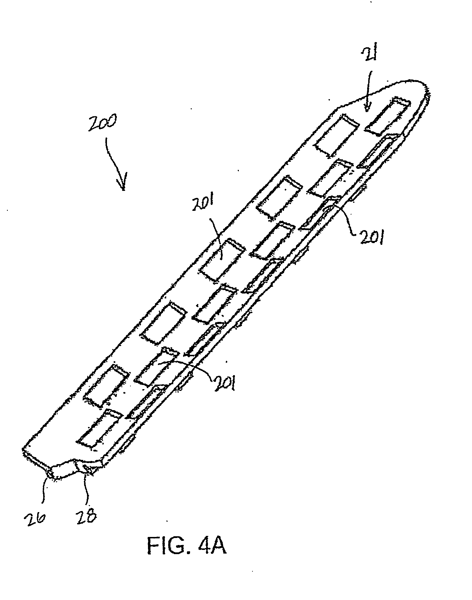

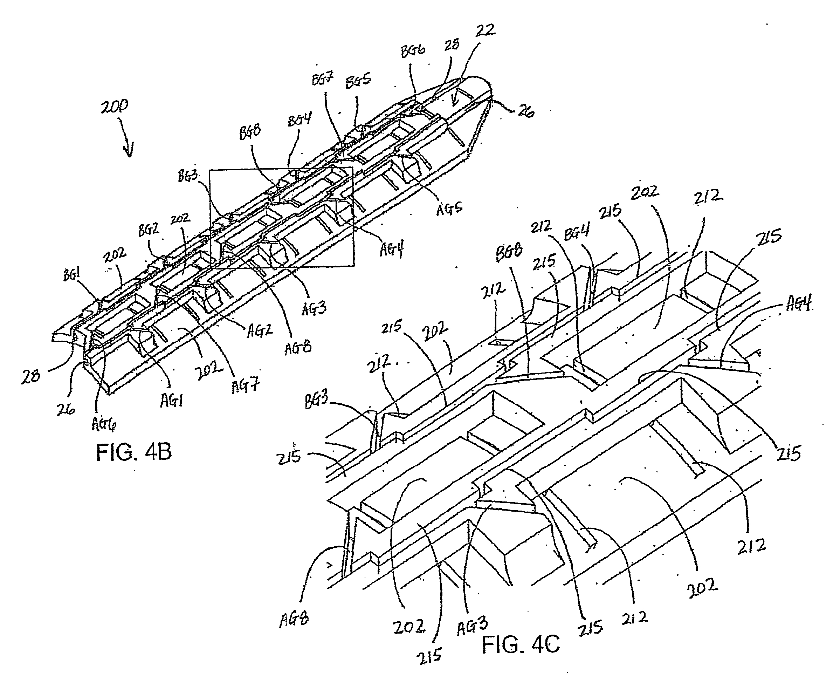

Figure 1A further illustrates, with a dashed line, a border of an insulative carrier 200 (Figures 4A-D), preferably formed from a flexible polymer, for example, silicone rubber which may have a polyester mesh panel embedded therein. According to the illustrated embodiment, carrier 200 is coupled to an insulative layer 170 to form body 101, and layer 170 is preferably formed from a material similar to that which forms carrier 200. Figure 1B further illustrates a first side 21 of carrier 200 corresponding to a side of body 101 from which electrodes AE1-8, BE1-8 may protrude, as illustrated; alternately, electrodes AE1-8, BE1-8 may be flush with first side 21 or recessed within first side 21. Insulative layer 170 is shown extending over a second side 22 (Figure 4B) of carrier 200 to form an opposite side 172 of body 101, and further extends about distal portions of bodies A,B to form proximal portion 151, for example, as described below in conjunction with Figure 5. According to embodiments of the present invention, each electrode AE1-8, BE1-8 is a portion 413 of a conductive component E, preferably formed from a 90/10 Platinum/iridium alloy, various embodiments of which are described in conjunction with Figures 2A-B, 3A-C, 6A-B and 7A-B.

Figures 2A-B illustrate electrode portion 413 of component E including an outward facing contact surface 43 and an inward facing surface 41; a pair of tabs 42, 44 extend from electrode portion 413 and are adapted to extend through carrier 200 in order to couple component E thereto. Figure 2A further illustrates each tab 42, 44 including a projection 420, 440, respectively. According to some embodiments of the present invention, component E is formed, for example, by stamping, such that portion 413, tabs 42, 44 and projections 420, 440 are approximately co-planar with one another, for example as shown in Figure 2B; then, tabs 42, 44 and projections 420, 440 are folded or bent away from portion 413 into the configuration illustrated in Figure 2A. Creases or indentations, for example, as illustrated by dashed lines in Figure 2B, may be formed in tabs 42, 44 in order to guide subsequent folding or bending. Alternately, component E may be provided as illustrated in Figure 2A by a single forming step, for example, machining or any other suitable forming method known to those skilled in the art. According to exemplary embodiments of the present invention, electrode portion 413 has a length, from tab 42 to tab 44, of approximately 0.185 inch (4.699 mm), and a width of approximately 0.086 inch (2,1844 mm); and tabs 42, 44 each have a thickness of approximately 0.005 inch (0.127 mm) and a length, from electrode portion 413 to projections 420, 440, respectively, of approximately 0.094 inch (2,3876 mm); and projections each have a length of approximately 0.028 inch (0.7112 mm).

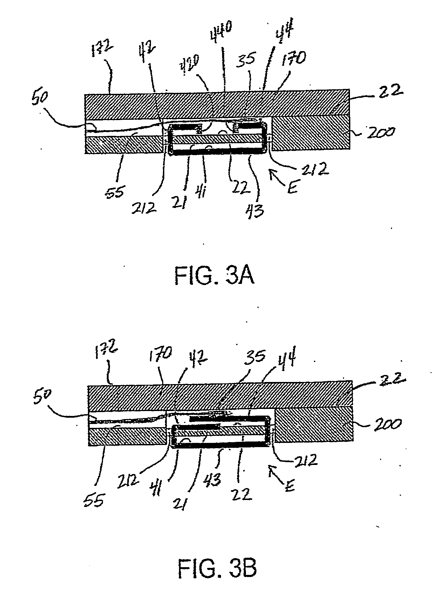

Figure 3A is a section view, through section line D-D of Figure 1A, wherein component E is coupled to carrier 200, according to some embodiments. Figure 3A illustrates each of tabs 42, 44, having been inserted through openings 212 of carrier 200, extending to second side 22 of carrier 200 where tabs 42, 44 are bent toward a surface of second side 22, such that tabs 42, 44 extend along the surface of second side 22, opposite inward facing surface 41 of the electrode portion that is disposed against a surface of first side 21 of carrier 200, and projections 420, 440 extend toward the surface of side 22. Pre-formed openings, for example, openings 212, may be sized to accommodate tabs 42, 44 including projections 420, 440, or any other tab cross-section for that matter; or, carrier 200 may be stretched to widen the pre-formed openings for insertion of tabs 42, 44 including projections 420, 440, or tabs having larger cross-sections that the openings. According to alternate embodiments, tabs 42, 44 do not include projections 220, 240. Furthermore, it should be noted that embodiments of the present invention need not include pre-formed openings, for example, openings 212 in carrier 200 for tabs 42, 44 to pass through, since tabs 42, 44 may form the openings by piercing carrier 200 upon insertion therethrough.

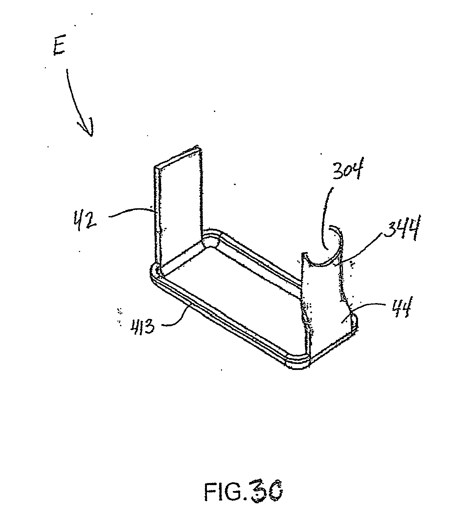

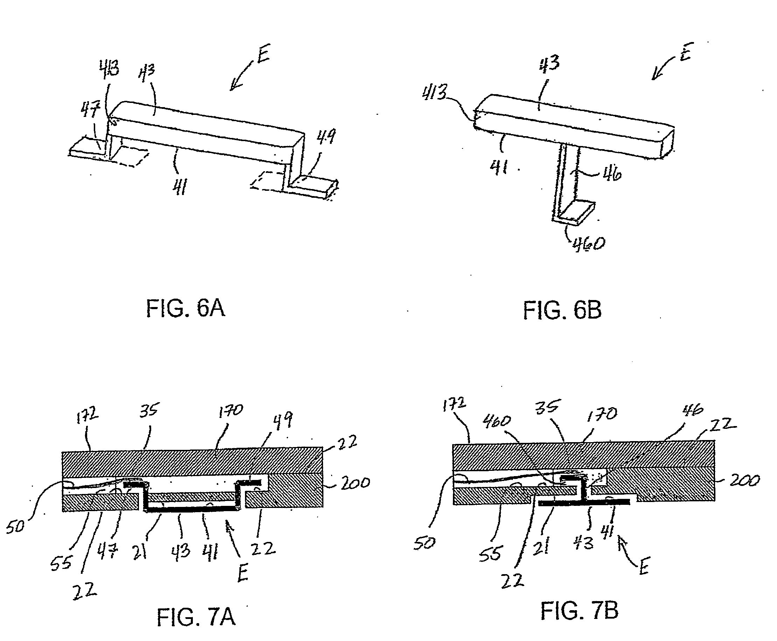

Referring back to Figure 2B, a length L42, L44 of each tab 42, 44, respectively, is indicated. According to further alternate embodiments of the present invention, length L44 is greater than length L42 such that when tabs 42, 44 are bent toward the surface of second side 22, tab 44 overlaps tab 42, as illustrated in Figure 3B. According to an exemplary embodiment wherein tabs overlap, a length of electrode portion 413, between tabs 42, 44 is approximately 0.185 inch (4.699 mm), length L44 of tab 44 is approximately 0.195 inch (4.953 mm), and length L42 of tab 42 is approximately 0.167 inch (4.2418 mm). (Another alternate embodiment of component E is described below, in conjunction with Figure 3C. Further embodiments of component E coupled to carrier 200, which generally correspond to additional alternate embodiments of component E, shown in Figures 6A-B, are shown in Figures 7A-B, and are also described below.)

According to the embodiments illustrated in Figures 3A-B, the surface of the first side 21, against which surface 41 of electrode portion 413 is disposed, is recessed; and, with reference to Figure 4A, it may be seen that first side 21 of carrier 200 includes a recess 201 for each electrode portion 413, for example electrodes AB1-8 and BE1-8 (Figure 1A). It should be noted that embodiments of the present invention need not include recesses 201. Figures 3A-B further illustrate a conductor 50 coupled to tab 44 of component E by a joint 35, which may be any suitable type of joint known to those skilled in the art, for example, a weld or a crimp or a combination thereof wherein a sleeve is crimped to conductor 50 and then the crimped sleeve is welded to tab 44. Although Figures 3A-B show joints 35 formed on a surface of tab 44 that faces away from second side 22, some alternate embodiments include joints formed along a surface of a tab that faces toward second side 22 of carrier 200, when the tab is bent to couple the corresponding component E to carrier 200. According to one alternate embodiment of component E, tab 44 is pre-formed to accommodate crimping of conductor 50 thereto, for example as illustrated in Figure 3C. Figure 3C shows tab 44 including a curved portion 344 forming a groove 304, into which groove 304 conductor 50 may be inserted for crimping therein, either prior to, or following, the bending of tab 44 to couple component E to carrier 200. It should be noted that, although tab 42 is shown, in Figure 3C, without a similar a pre-formed curve, tab 42 may also be pre-formed as tab 44.

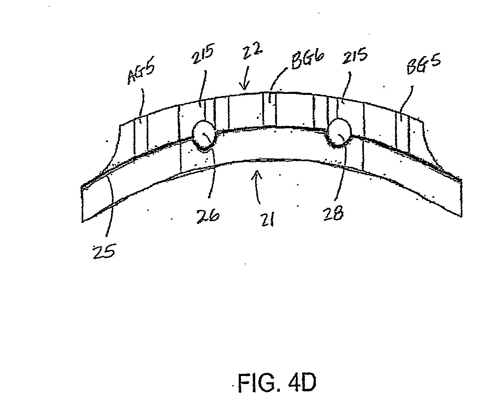

Referring back to Figures 3A-B, conductor 50 is shown extending from joint 35 into a pre-formed channel 55 along second side 22 of carrier. With reference to Figure 4B, an entirety of second side 22 of carrier 200, according to one embodiment, may be seen. Figure 4B illustrates a plurality of pre-formed channels AG1-8 and BG1-8 (each corresponding to channel 55 illustrated in Figures 3A-B and 7A-B); each channel AG1-8 and BG1-8 is shown extending from a corresponding pre-formed recess 202 that accommodates a pair of tabs 42, 44. According to the illustrated embodiment, channels AG1-8 and BG1-8 direct each conductor 50 from the corresponding joint 35 (Figures 3A and 5) into either a first or second longitudinally extending pre-formed channel 26, 28. Figure 4B shows first longitudinally extending pre-formed channel 26 extending from each of channels AG 1-8, and second longitudinally extending pre-formed channel 28 extending from each of channels BG1-8. Figure 4C is an enlarged view of a portion of carrier 200 enclosed in the box shown in Figure 4B. With reference to Figure 4B-C, it may be seen that a series of flaps 215 extend in a zipper-like fashion over each of channels 26 and 28; conductors 50 may be pressed across flaps 215 into channels 26,28 and then held in place by flaps 215. Figure 4D is a distal end view of carrier 200 showing flaps 215 extending over channels 26 and 28.

Figure 4D further illustrates a panel 25, for example, formed from a polyester mesh material, extending just beneath recessed surfaces of second side 22 of carrier 200. According to some alternate embodiments, panel 25 extends just beneath an exterior surface of first side 21. According to preferred embodiments of the present invention, panel 25 is integrally formed with carrier 200, having a foot print similar to that of carrier 200, for example, as defined by the dashed lines in Figure 1A, to provide some additional tear resistance to carrier 200.

Figure 5A is a plan view of an electrode assembly 30, incorporating carrier 200, wherein conductors 50 (Figures 3A-B and 7A-B) are designated as a first plurality A1-8 and a second plurality B1-8, and dashed lines indicate a border of insulative layer 170, which has been made transparent in order to show a routing of conductors A1-8 and B1-8. Figure 5A illustrates both of channels 26 and 28 extending approximately parallel to a longitudinal axis 300 of carrier 200, channel 26 between columns 12 and 13 to direct conductors A1-8 into tubular body A, and channel 28 between columns 13 and 14 to direct conductors B1-8 into tubular body B. According to the illustrated embodiment, each joint 35 is provided with some strain-relief against longitudinal loading by pre-formed channels AG1-8 and BG1-8 (Figure 4B), which direct conductors A1-8 and B1-8, at an angle to longitudinal axis 300, away from respective joints 35, and into respective longitudinal channels 26, 28.

Figure 5A further illustrates tubular bodies A and B each including a distal portion AX and BX, respectively; and, with reference to Figure 5A in conjunction with Figure 1A, it may be appreciated that portions AX and BX extend into proximal portion 151 of body 101 formed by insulative layer 170; layer 170 may further extend into pre-formed apertures 33 formed in sidewalls of portions AX, BX. It should be understood that portions AX and BX form extensions of the one or more longitudinal channels, or lumens, of tubular bodies A, B so that conductors A1-8 and B1-8, respectively may pass proximally therethrough from channels 26, 28. Figure 5A further illustrates each portion AX, BX including a curved segment 39, wherein segments 39 are longitudinally offset from one another, and an apex of each segment 39 is approximately aligned with one another in close proximity to longitudinal axis 300. The illustrated u-shape of each segment 39, and the arrangement of segments 39 may facilitate compact coupling of segments 39 to carrier 200, within layer 170; however, the scope of the present invention is not so limited, and any suitable curved shape and arrangement can be incorporated in alternate embodiments. Curved segments 39 may provide addition strain-relief and minimize flex-fatigue for the conductors A1-8, B1-8, and may further prevent longitudinal forces from dislodging electrode assembly 30 from an implanted location. According to exemplary embodiments, tubular bodies A, B are each formed from a polyurethane material and insulative layer 170 is formed from a silicone material; because silicone and polyurethane materials may not bond to one another, the mechanical interlocking between layer 170 and tubular bodies A,B, at apertures 33 and along curve segments 39, may provide additional structural integrity to assembly 30, for these exemplary embodiments.

Figures 5B-C are plan views of alternative electrode assemblies 30' and 30", respectively. Figures 5B-C illustrate portions AX and BX, of tubular bodies A and B, respectively, within insulative layer 170, including no curved segments, such as segments 39 illustrated in Figure 5A. According to the embodiment of Figure 5B, first plurality of conductors A1-8 are formed in a bend between channel 26 and portion AX, and second plurality of conductors B 1-8 are likewise formed in a bend between channel 28 and portion BX. According to the embodiment of Figure 5C, first plurality of conductors A1-8 cross over from channel 26 to enter tubular body B and second plurality of conductors B1-8 cross over from channel 28 to enter tubular body A. Conductors A1-8 and B 1-8 may be held in the illustrated configurations by additional channels formed in carrier 200 or may be positioned as such prior to molding of insulative layer 170 thereover. It should be noted that embodiments of the present invention may further include those in which conductors A1-8, B1-8 extend in approximately straight paths from channels 26 and 28 into respective tubular bodies A, B. It should be noted that each conductor of pluralities A1-8 and B1-8 are electrically isolated from one another by an insulating jacket extending around each conductor.

Although Figures 5A-C illustrates a preferred embodiment wherein joints 3 5 are made between a distally located tab of each component E and the corresponding conductor, the scope of the present invention is not limited to any particular orientation of tabs 42 and 44, with respect to proximal and distal ends 51 and 53, respectively, of carrier 200; for example, some alternate embodiments include conductive components E oriented in carrier such that tabs 42, 44 are disposed on opposite sides of a longitudinal axis of the corresponding column 12, 13, 14. According to further alternate embodiments, joints 35 are made with the proximally located tabs shown in Figure 5A; or joint 35 may be made with a centrally located tab, for example, tab 46 shown in Figure 7B for the embodiment of component E illustrated in Figure 6B.

Figures 6A-B are perspective views of conductive component E, according to additional alternate embodiments of the present invention; and Figures 7A-B are section views taken through section line D-D of Figure 1A, according to embodiments that generally correspond to the embodiments of components E illustrated in Figures 6A-B, respectively. Figure 6A illustrates component E including tabs 47 and 49 extending outward such that tabs 47, 49, when component E is coupled to carrier 200, extend along a surface of second side 22 of carrier 200, which is offset from inward facing surface 41 of electrode portion 413, for example as illustrated in Figure 7A. According to the illustrated embodiment, tabs 47, 49 are pre-formed prior to insertion through carrier 200, however, according to an alternate embodiment, tabs 47, 49 are bent outward after insertion through carrier 200. Dashed lines in Figure 6A illustrate yet another alternative embodiment of component E, wherein tabs 47, 49 extend both inward, toward one another, and outward. Figure 6B, as previously described, illustrates component E including centrally located tab 46 extending from electrode portion 413; tab 46 is shown including a projection 460 which extends toward a surface of second side 22 of carrier when tab 46 is bent, as illustrated in Figure 7B. Of course tab 46 need not include projection 460, and, according to alternate embodiments, tab 46 may be pre-formed, as shown in Figure 7B, prior to insertion through carrier 200. Those skilled in the art will recognize that various numbers and configurations of tabs, extending from electrode portion 413 of component E, may be employed within the scope of the present invention.

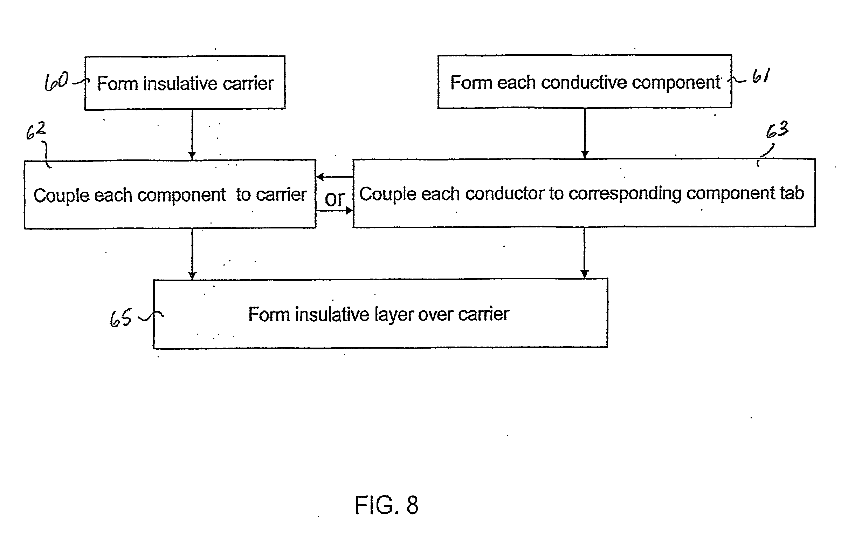

Figures 8 is a flow chart outlining alternate methods of the present invention for making inventive electrode assemblies. Figure 8 outlines methods wherein forming an insulative carrier, for example carrier 200, is one initial step 60 and forming each conductive component, for example, component E, is another initial step 61. After each component and the carrier is formed, a conductor is coupled to a tab of the corresponding component, per step 63, either before or after each component is coupled to the carrier, per step 62. In a latter step 65, an insulative layer is formed over the carrier to encapsulate each component tab and conductor. According to preferred methods, the carrier is formed (step 60) via a molding process, for example, transfer or injection molding, and a polymer mesh panel, for example, panel 25 (Figure 3D), is integrated into the carrier by inserting the panel into a mold prior to completing the molding process. A mold, which is used by some methods, includes features to form openings through the carrier and channels and recesses on first and second sides of the carrier, which openings, channels and recesses are described above in conjunction with carrier 200. According to some alternate methods, a process secondary to molding may be used to form channels and/or recesses, for example, by other types of thermal forming known to those skilled in the art, or by cutting or abrading methods, or by bonding additional layers to the molded carrier. For those embodiments of the carrier which include pre-formed openings, for example, openings 212, corresponding openings may be formed in the mesh panel prior to placing the panel in the mold, the panel openings being aligned with mold features for forming the openings of carrier, when the panel is placed.

Forming each conductive component (step 61) may be performed as described in conjunction with Figures 2A-B, and coupling each component to the carrier (step 62) is performed by inserting one or more tabs of the component through the carrier such that an electrode portion of the component is disposed on the first side of the carrier and the one or more tabs are disposed on the second side of the carrier to secure the component to the carrier, for example as illustrated in Figures 3A-B and 7A-B. The one or more tabs may be bent, after insertion through the carrier, or pre-formed in a bent configuration, prior to insertion through the carrier, such that bending the tabs after insertion through the carrier is not required to secure the component to the carrier. According to a preferred method of the present invention, the tabs of all of components E are bent or folded simultaneously after having been inserted through the carrier. All the tabs may be simultaneously folded by a plate that is pressed down toward the second side of the carrier while the components are supported, for example, along contact surfaces 43 of electrode portions 413.

According to some methods, after each component is coupled to the carrier (step 62), each conductor is coupled to the corresponding component tab, per step 63. According to some alternate methods, each conductor is coupled to the corresponding component tab (step 63) prior to coupling each component to the carrier (step 62). If step 62 follows step 63, a length of the conductor may be passed through the carrier ahead of the tab to couple the component to the carrier, or, the electrode portion of the component may be passed through the carrier, ahead of the tab. Although not shown in the outline of Figure 8, each conductor may be routed in grooves on the second side of the carrier either before or after coupling each conductor to the corresponding component tab. As previously described in conjunction with Figures 4B-D, Figure 5A, and method step 60, insulative carriers, according to some preferred embodiments of the present invention, include pre-formed channels which are suitable for routing conductors along the carrier to insulative tubular bodies and have features to hold conductors in place on the carrier while the insulative layer is formed over the carrier (step 65). Such channels may further provide some strain-relief for the coupling between each conductor and the corresponding component tab. Coupling each conductor to the corresponding component tab, per step 63, may be performed by any of the methods previously described, for example, by crimping, welding (e.g. laser or resistance), or a combination thereof, or by any other suitable method known to those skilled in the art.

Once each component and corresponding conductor, coupled thereto, are mounted or coupled to the carrier, an insulative layer may be formed, per step 65, over the side of the carrier on which the component tabs and conductors are disposed. As previously described, the insulative layer may further surround a portion of one or more elongate tubular bodies into which each conductor has been inserted, for example, portions AX, BX of tubular bodies A, B shown in Figures 5A-C. Each conductor may have been inserted into the corresponding tubular member at any point, before step 65, in the method outlined by Figure 8, that is, before or after any of steps 60, 61, 62, and 63. According to some methods of the invention, prior to insertion of each conductor into the corresponding tubular body, each tubular body is thermal formed to include a curved segment, for example segment 39, shown in Figure 5A, and may also be perforated to include an aperture, for example aperture 33, also shown in Figure 5A. Forming the insulative layer (step 65) is preferably performed by an over-molding process, for example, either by injection molding or transfer molding. According to the over-molding process, the carrier on which each electrode and corresponding conductor are mounted, along with each tubular body, in which each corresponding conductors has been inserted, are placed in a mold, and an insulative material, preferably silicone rubber, is injected into the mold to cover each conductor and component tab, for example, along second side 22 of carrier 200, and to surround each tubular body, for example, as illustrated in Figures 1A and 5A-C.

In the foregoing detailed description, the invention has been described with reference to specific embodiments. However, it may be appreciated that various modifications and changes can be made without departing from the scope of the invention as set forth in the appended claims.

## Claims

1. A medical electrical lead electrode assembly, comprising:
an insulative body including an insulative layer (170) and a pre-formed carrier (200) coupled to the insulative layer, the carrier including a first side (21), a second side (22) and a pre-formed channel on the second side, the channel including a first section (26, 28), extending approximately parallel to a longitudinal axis (300) of the carrier, and at least one second section (BG1-8, AG1-8, AX, BX), extending at an angle to the longitudinal axis and approximately co-planar with the first section of the channel;
at least one conductive component (E) including an electrode portion (413) disposed on the first side of the carrier and a tab (42, 44, 46, 47, 49) extending away from the electrode portion and through the carrier to the second side of the carrier; and
a conductor (50, A1-8, B1-8) disposed in the channel and coupled to the tab of the component;
wherein the conductor, the first section of the channel, the at least one second section of the channel, and the tab of the at least one component are overlaid with the insulative layer;
wherein the first section of the channel comprises a groove formed in the second side of the carrier;
**characterized in that**
the carrier further includes a pre-formed flap (215) that extends over the first section of the channel and the conductor disposed therein; and
the pre-formed flap is also overlaid with the insulative layer.

2. The assembly of claim 1, wherein the at least one second section of the pre-formed channel extends between the tab and the first section of the channel.

3. The assembly of claim 1, further **characterized in that**:
the pre-formed channel further includes a third section extending proximally from the insulative body:
the at least one second section (AX,BX) of the channel extends between the first section of the channel and the third section of the channel; and
the at least one second section and the third section of the channel comprise a tubular member (A, B).

4. The assembly of claim 3, further **characterized in that** the tubular member includes an aperture (33) formed in a sidewall thereof and the insulative layer extends through the aperture.

5. The assembly of claim 3, further **characterized in that** the at least one second section includes a curved segment (39).

6. The assembly of claim 1, wherein:
the at least one second section of the channel comprises a plurality of second sections (BG1-BG8, AG1-AG8) spaced apart along a length of the first section of the channel;
the at least one conductive component comprises a plurality of components arranged in at least one column (12, 13, 14), the at least one column of conductive components extending approximately parallel to the longitudinal axis of the carrier; and
the conductor comprises a plurality of conductors;
each of the plurality of conductors is coupled to the tab of a corresponding component of the plurality of components;
each second section of the plurality of second sections of the channel extends between the tab of a corresponding component of the plurality of components and the first section of the channel; and
each conductor of the plurality of conductors is disposed in a corresponding second section of the plurality of second sections of the channel.

7. The assembly of claim 6, wherein the at least one column of conductive components comprises a first column of components and a second column of components, the first and second columns disposed on either side of the first section of the channel.

8. The assembly of claim 1, wherein:
the pre-formed channel is a first channel and the carrier further includes a second pre-formed channel, the second channel including a first section, extending approximately parallel to the longitudinal axis of the carrier, and a second section, extending at an angle to the longitudinal axis, approximately co-planar with the first section of the second channel;
the at least one conductive component comprises a first column (12) of conductive components disposed between the first and second channels, a second column (13) of conductive components disposed alongside the first channel and opposite the first column of conductive components, and a third column (14) of conductive components disposed alongside the second channel and opposite the first column of conductive components, each column of conductive components extending approximately parallel to the longitudinal axis of the carrier;
the conductor comprises a first plurality of conductors (A1-8);
each of the first plurality of conductors is coupled to the tab of a corresponding component of the first and second columns of components; and
further comprising a second plurality of conductors (B1-8);
wherein each of the second plurality of conductors is disposed in the second channel and coupled to the tab of a corresponding component of the first and third columns of components;
further **characterized in that**:
the first section of the second channel comprises a groove formed in the carrier;
the carrier further includes another pre-formed flap (215) that extends over the first section of the second channel and each of the second plurality of conductors disposed therein; and
the other pre-formed flap that extends over the first section of the second channel is also overlaid with the insulative layer.

9. The assembly of claim 8, further **characterized in that**:
each of the first and second pre-formed channels further include a third section extending proximally from the insulative body;
the second section of the first channel extends between the first and third sections thereof;
the second section of the second channel extends between the first and third sections thereof; and
the second and third sections of the first channel and the second and third sections of the second channel each comprise a tubular member (A, B).

10. The assembly of claim 9, further **characterized in that** each of the tubular members includes an aperture (33) formed in a sidewall thereof and the insulative layer extends through the aperture.

11. The assembly of claim 9, further **characterized in that** each of the second sections of the first and second channels includes a curved segment (39).

## Patentansprüche

1. Elektrodenanordnung für eine medizinische elektrische Leitung, die aufweist:
einen isolierenden Körper mit einer isolierenden Schicht (170) und einem vorgeformten Träger (200), der mit der isolierenden Schicht verbunden ist, wobei der Träger eine erste Seite (21), eine zweite Seite (22) und einen vorgeformten Kanal auf der zweiten Seite aufweist, wobei der Kanal einen ersten Abschnitt (26, 28), der sich in etwa parallel zur Längsachse (300) des Trägers erstreckt, und wenigstens einen zweiten Abschnitt (BG1-8, AG1-8, AX, BX), der sich in einem Winkel zur Längsachse erstreckt und in etwa mit dem ersten Abschnitt des Kanals koplanar ist, beinhaltet;
wenigstens ein leitendes Element (E) das einen Elektrodenteil (413), der auf der ersten Seite des Trägers angeordnet ist, und einen Streifen bzw. Tab (42, 44, 46, 47, 49), der sich von dem Elektrodenteil weg und durch den Träger zur zweiten Seite des Trägers erstreckt, beinhaltet; und
einen Leiter (50, A1-8, B1-8), der in dem Kanal angeordnet und mit dem Tab der Komponente verbunden ist;
wobei der Leiter, der erste Abschnitt des Kanals, der wenigstens eine zweite Abschnitt des Kanals und der Tab der wenigstens einen Komponente mit der isolierenden Schicht überlagert bzw. überschichtet sind;
wobei der erste Abschnitt des Kanals eine in der zweiten Seite des Trägers ausgebildete Rinne bzw. Rille aufweist;
**dadurch gekennzeichnet, dass**
der Träger ferner eine vorgeformte Lasche (215), die sich über den ersten Abschnitt des Kanals und den hierin angeordneten Leiter erstreckt, aufweist; und
die vorgeformte Lasche auch durch die isolierende Schicht überlagert ist.

2. Anordnung nach Anspruch 1, bei der wenigstens ein zweiter Abschnitt des vorgeformten Kanals sich zwischen dem Tab und dem ersten Abschnitt des Kanals erstreckt.

3. Anordnung nach Anspruch 1, ferner **dadurch gekennzeichnet, dass**:
der vorgeformte Kanal ferner einen dritten Abschnitt beinhaltet, der sich proximal des isolierenden Körpers erstreckt;
der wenigstens eine erste Abschnitt (AX, BX) des Kanals sich zwischen dem zweiten Abschnitt des Kanals und dem dritten Abschnitt des Kanals erstreckt; und
der wenigstens eine zweite Abschnitt und der dritte Abschnitt des Kanals ein rohrförmiges Element (A, B) aufweisen.

4. Anordnung nach Anspruch 3, ferner **dadurch gekennzeichnet, dass** das rohrförmige Element eine Öffnung (33), die in einer Seitenwand desselben ausgeformt ist, aufweist, und dass sich die isolierende Schicht durch die Öffnung erstreckt.

5. Anordnung nach Anspruch 3, ferner **dadurch gekennzeichnet, dass** der wenigstens eine zweite Abschnitt ein gebogenes bzw. gekrümmtes Segment (39) aufweist.

6. Anordnung nach Anspruch 1, bei der:
der wenigstens eine zweite Abschnitt des Kanals eine Anzahl von zweiten Abschnitten (BG1-BG8, AG1-AG8) aufweist, die entlang einer Länge des ersten Abschnitts des Kanals voneinander beabstandet sind;
die wenigstens eine leitende Komponente eine Anzahl von Komponenten aufweist, die in wenigstens einer Spalte bzw. Säule (12, 13, 14) angeordnet sind, wobei die wenigstens eine Spalte leitender Elemente sich in etwa parallel zur Längsachse des Trägers erstreckt; und
der Leiter eine Anzahl von Leitern aufweist;
jeder aus der Anzahl von Leitern mit dem Tab einer entsprechenden Komponente aus der Anzahl von Komponenten verbunden ist;
jeder zweite Abschnitt aus der Anzahl von zweiten Abschnitten des Kanals sich zwischen dem Tab einer entsprechenden Komponente aus der Anzahl von Komponenten und dem ersten Abschnitt des Kanals erstreckt; und
jeder Leiter aus der Anzahl von Leitern in einem entsprechenden zweiten Abschnitt aus der Anzahl von zweiten Abschnitten des Kanals angeordnet ist.

7. Anordnung nach Anspruch 6, bei der die wenigstens eine Spalte leitender Elemente wenigstens eine erste Spalte von Komponenten und eine zweite Spalte von Komponenten beinhaltet, wobei die ersten und zweiten Spalten auf beiden Seiten des ersten Abschnitts des Kanals angeordnet sind.

8. Anordnung nach Anspruch 1, bei der:
der vorgeformte Kanal ein erster Kanal ist und der Träger ferner einen zweiten vorgeformten Kanal beinhaltet, wobei der zweite Kanal einen ersten Abschnitt, der sich in etwa parallel zur Längsachse des Trägers erstreckt, und einen zweiten Abschnitt, der sich in einem Winkel zu der Längsachse, in etwa koplanar mit dem ersten Abschnitt des zweiten Kanals, erstreckt, beinhaltet;
die wenigstens eine leitende Komponente eine erste Spalte (12) leitender Komponenten, die zwischen den ersten und zweiten Kanälen angeordnet sind, eine zweite Spalte (13) leitender Komponenten, die entlang dem ersten Kanal und gegenüber der ersten Spalte leitender Komponenten angeordnet sind, und eine dritte Spalte (14) leitender Komponenten, die entlang dem zweiten Kanal und gegenüber der ersten Spalte leitender Elemente angeordnet sind, beinhaltet, wobei jede Spalte leitender Komponenten sich in etwa parallel zur Längsachse des Trägers erstreckt;
der Leiter eine erste Anzahl von Leitern (A1-8) aufweist;
jeder aus der ersten Anzahl von Leitern mit dem Tab einer entsprechenden Komponente der ersten und zweiten Spalten von Komponenten verbunden ist; und
ferner mit einer zweiten Anzahl von Leitern (B1-8);
wobei jeder aus der zweiten Anzahl von Leitern in dem zweiten Kanal angeordnet und mit dem Tab einer entsprechenden Komponente aus den ersten und dritten Spalten von Komponenten verbunden ist;
ferner **dadurch gekennzeichnet, dass**:
der erste Abschnitt des zweiten Kanals eine in dem Träger ausgebildete Rille aufweist;
der Träger ferner eine andere vorgeformte Lasche (215) beinhaltet, die sich über den ersten Abschnitt des zweiten Kanals und jeden aus der zweiten Anzahl von hierin angeordneten Leitern erstreckt; und
die andere vorgeformte Lasche, die sich über den ersten Abschnitt des zweiten Kanals erstreckt, ebenso durch die isolierende Schicht überlagert ist.

9. Anordnung nach Anspruch 8, ferner **dadurch gekennzeichnet, dass**:
sowohl der erste als auch der zweite vorgeformte Kanal ferner einen dritten Abschnitt beinhalten, der sich proximal des isolierenden Körpers erstreckt;
der zweite Abschnitt des ersten Kanals sich zwischen den ersten und dritten Abschnitten desselben erstreckt;
der zweite Abschnitt des zweiten Kanals sich zwischen den ersten und dritten Abschnitten desselben erstreckt; und
der zweite und dritte Abschnitt des ersten Kanals und der zweite und dritte Abschnitt des zweiten Kanals jeweils ein rohrförmiges Element (A, B) aufweist.

10. Anordnung nach Anspruch 9, ferner **dadurch gekennzeichnet, dass** jedes der rohrförmigen Elemente eine Öffnung (33), die in einer Seitenwand desselben ausgebildet ist, aufweist, und die isolierende Schicht sich durch die Öffnung erstreckt.

11. Anordnung nach Anspruch 9, ferner **dadurch gekennzeichnet, dass** jeder der zweiten Abschnitte der ersten und zweiten Kanäle ein gebogenes Segment (39) beinhaltet.

## Revendications

1. Ensemble d'électrodes de dérivation électriques médicales, comportant :
un corps isolant incluant une couche isolante (170) et un support préformé (200) couplé à la couche isolante, le support incluant un premier côté (21), un second côté (22) et un canal préformé sur le second côté, le canal incluant un premier tronçon (26, 28), s'étendant approximativement parallèle à un axe longitudinal (300) du support, et au moins un deuxième tronçon (BG1-8, AG1-8, AX, BX), s'étendant à un angle par rapport à l'axe longitudinal et approximativement coplanaire avec le premier tronçon du canal ;
au moins un composant conducteur (E) incluant une partie d'électrode (413) disposée sur le premier côté du support et une languette (42, 44, 46, 47, 49) s'étendant en s'éloignant de la partie d'électrode et à travers le support jusqu'au second côté du support ; et
un conducteur (50, A1-8, B1-8) disposé dans le canal et couplé à la languette du composant ;
dans lequel le conducteur, le premier tronçon du canal, le au moins un deuxième tronçon du canal, et la languette du au moins un composant sont recouverts de la couche isolante ;
dans lequel le premier tronçon du canal comporte une rainure formée dans le second côté du support ;
**caractérisé en ce que** le support inclut en outre un rabat préformé (215) qui s'étend au-dessus du premier tronçon du canal et du conducteur disposé dans celui-ci ; et
le rabat préformé est également recouvert de la couche isolante.

2. Ensemble selon la revendication 1, dans lequel le au moins un deuxième tronçon du canal préformé s'étend entre la languette et le premier tronçon du canal.

3. Ensemble selon la revendication 1, également **caractérisé en ce que** :
le canal préformé inclut également un troisième tronçon s'étendant à proximité du corps isolant ;
le au moins un deuxième tronçon (AX, BX) du canal s'étend entre le premier tronçon du canal et le troisième tronçon du canal ; et
le au moins un deuxième tronçon et le troisième tronçon du canal comportent un élément tubulaire (A, B).

4. Ensemble selon la revendication 3, **caractérisé en outre en ce que** l'élément tubulaire inclut une ouverture (33) formée dans une paroi latérale de celui-ci, et la couche isolante s'étend à travers l'ouverture.

5. Ensemble selon la revendication 3, **caractérisé de plus en ce que** le au moins un deuxième tronçon inclut un segment courbe (39).

6. Ensemble selon la revendication 1, dans lequel :
le au moins un deuxième tronçon du canal comporte une pluralité de deuxièmes tronçons (BG1-BG8, AG1-AG8) espacés les uns des autres le long d'une longueur du premier tronçon du canal ;
le au moins un composant conducteur comporte une pluralité de composants agencés dans au moins une colonne (12, 13, 14), la au moins une colonne de composants conducteurs s'étendant approximativement parallèle à l'axe longitudinal du support ; et
le conducteur comporte une pluralité de conducteurs ;
chacun de la pluralité de conducteurs est couplé à la languette d'un composant correspondant de la pluralité de composants ;
chaque deuxième tronçon de la pluralité de deuxièmes tronçons du canal s'étend entre la languette d'un composant correspondant de la pluralité de composants et le premier tronçon du canal ; et
chaque conducteur de la pluralité de conducteurs est disposé dans un deuxième tronçon correspondant de la pluralité de deuxièmes tronçons du canal.

7. Ensemble selon la revendication 6, dans lequel la au moins une colonne de composants conducteurs comporte une première colonne de composants et une seconde colonne de composants, les première et seconde colonnes étant disposées de chaque côté du premier tronçon du canal.

8. Ensemble selon la revendication 1, dans lequel :
le canal préformé est un premier canal et le support inclut en outre un second canal préformé, le second canal incluant un premier tronçon, s'étendant approximativement parallèle à l'axe longitudinal du support, et un deuxième tronçon, s'étendant à un angle par rapport à l'axe longitudinal, approximativement coplanaire avec le premier tronçon du second canal ;
le au moins un composant conducteur comporte une première colonne (12) de composants conducteurs disposée entre les premier et second canaux, une deuxième colonne (13) de composants conducteurs disposée à côté du premier canal et opposée à la première colonne de composants conducteurs, et troisième colonne (14) de composants conducteurs disposée à côté du second canal et opposée à la première colonne de composants conducteurs, chaque colonne de composants conducteurs s'étendant approximativement parallèle à l'axe longitudinal du support ;
le conducteur comporte une première pluralité de conducteurs (A1-8) ;
chacun de la première pluralité de conducteurs est couplé à la languette d'un composant correspondant des première et deuxième colonnes de composants ; et comportant en outre une seconde pluralité de conducteurs (B1-8) ;
dans lequel chacun de la seconde pluralité de conducteurs est disposé dans le second canal et couplé à la languette d'un composant correspondant des première et troisième colonnes de composants ;
**caractérisé en outre en ce que** :
le premier tronçon du second canal comporte une rainure formée dans le support ;
le support inclut également un autre rabat préformé (215) qui s'étend au-dessus du premier tronçon du second canal et de chacun de la seconde pluralité de conducteurs disposés dans celui-ci ; et
l'autre rabat préformé qui s'étend au-dessus du premier tronçon du second canal est également recouvert de la couche isolante.

9. Ensemble selon la revendication 8, également **caractérisé en ce que** :
chacun des premier et second canaux préformés inclut en outre un troisième tronçon s'étendant de manière proximale à partir du corps isolant ;
le deuxième tronçon du premier canal s'étend entre les premier et troisième tronçons de celui-ci ;
le deuxième tronçon du second canal s'étend entre les premier et troisième tronçons de celui-ci ; et
les deuxième et troisième tronçons du premier canal et les deuxième et troisième tronçons du second canal comportent chacun un élément tubulaire (A, B).

10. Ensemble selon la revendication 9, également **caractérisé en ce que** chacun des éléments tubulaires inclut une ouverture (33) formée dans une paroi latérale de celui-ci et la couche isolante s'étend à travers l'ouverture.

11. Ensemble selon la revendication 9, **caractérisé de plus en ce que** chacun des deuxièmes tronçons des premier et second canaux inclut un segment courbe (39).
